(19) Europäisches Patentamt
European Patent Office
Office européen des brevets

(11) Veröffentlichungsnummer: **0 075 234**
**B1**

(12) **EUROPÄISCHE PATENTSCHRIFT**

(45) Veröffentlichungstag der Patentschrift: **12.09.84**

(51) Int. Cl.[3]: **C 07 C 69/732,** C 07 C 67/31

(21) Anmeldenummer: **82108420.9**

(22) Anmeldetag: **13.09.82**

# (54) Neue Acyloxyalkadiencarbonsäureester und Verfahren zu ihrer Herstellung.

(30) Priorität: **23.09.81 DE 3137801**

(43) Veröffentlichungstag der Anmeldung:
**30.03.83 Patentblatt 83/13**

(45) Bekanntmachung des Hinweises auf die Patenterteilung:
**12.09.84 Patentblatt 84/37**

(84) Benannte Vertragsstaaten:
**CH DE FR GB IT LI**

(56) Entgegenhaltungen:
**Keine Entgegenhaltungen**
**KEINE**

(73) Patentinhaber: **BASF Aktiengesellschaft,**
**Carl-Bosch-Strasse 38, D-6700 Ludwigshafen (DE)**

(72) Erfinder: **Fischer, Rolf, Dr., Bergstrasse 98,**
**D-6900 Heidelberg (DE)**
Erfinder: **Weitz, Hans-Martin, Dr., Auf dem Koeppel 40,**
**D-6702 Bad Duerkheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 075 234 B1

**Beschreibung**

Die Erfindung betrifft neue Acyloxyalkadiencarbonsäureester und ein Verfahren zu ihrer Herstellung.

Die neuen Acyloxyalkadiencarbonsäureester haben die allgemeine Formel

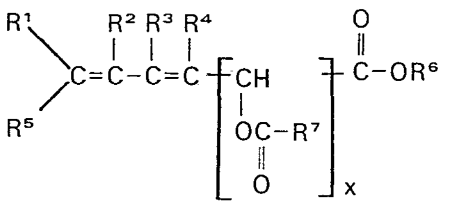

(I)

in der $R^1$ ein Wasserstoffatom, einen Kohlenwasserstoffrest mit 1 bis 5 C-Atomen oder einen $R^6$-O-CO-Rest, die Reste $R^2$ und $R^3$ Wasserstoffatome oder Kohlenwasserstoffreste mit 1 bis 5 C-Atomen, $R^4$ ein Wasserstoffatom oder einen Kohlenwasserstoffrest mit 1 bis 5 C-Atomen, jedoch für den Fall, dass x für O steht, ein Wasserstoffatom, $R^5$ ein Wasserstoffatom oder für den Fall, dass x für O steht, einen

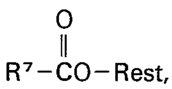

$R^6$ einen Kohlenwasserstoffrest mit 1 bis 15 C-Atomen, $R^7$ ein Wasserstoffatom oder einen Kohlenwasserstoffrest mit 1 bis 5 C-Atomen und x entweder 0 oder 1 bedeuten.

Als Kohlenwasserstoffreste enthalten die Verbindungen der Formel I z.B. Alkyl-, Alkenyl- oder Alkadienylreste, wie $CH_3$-, $C_2H_5$-, $C_3H_7$-, n-$C_4H_9$-, sek.-Butyl-, i-Butyl-, tert.-Butyl-, $CH_2=CH$-, $CH_3$-$CH=CH$-, $CH_2=CH$-$CH=CH$-$CH_2$-, n-$C_5H_{11}$-, n-$C_{10}H_{21}$-, n-$C_{12}H_{25}$- oder n-$C_{15}H_{31}$-Reste. Kohlenwasserstoffreste mit bis zu 15 C-Atomen können auch Cycloalkyl-, Aryl- oder Benzylreste sein.

Von besonderem technischem Interesse sind solche Verbindungen der Formel I, in denen die Reste $R^1$ bis $R^4$ und $R^6$ bis $R^7$ Wasserstoffatome oder Alkylreste mit 1 bis 3 C-Atomen bedeuten.

Die neuen Acyloxyalkadiencarbonsäureester der Formel I werden dadurch hergestellt, dass man Diene der Formel II

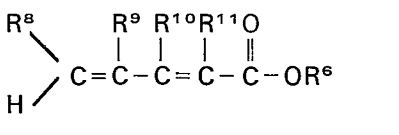

(II)

in der $R^8$ ein Wasserstoffatom, einen Kohlenwasserstoffrest mit 1 bis 5 C-Atomen oder einen $R^6$-O-CO-Rest und die Reste $R^9$, $R^{10}$ und $R^{11}$ Wasserstoffatome oder Kohlenwasserstoffreste mit 1 bis 5 C-Atomen bedeuten, in Gegenwart von Katalysatoren, die Palladium, Platin oder Salze dieser Metalle enthalten, und von Sauerstoff mit Carbonsäuren der Formel $R^7COOH$ behandelt.

Die Reaktion kann für den Fall der Umsetzung von Sorbinsäuremethylester (2,4-Hexadiencarbonsäuremethylester) mit Essigsäure und Sauerstoff durch die folgenden Formeln wiedergegeben werden 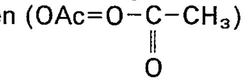

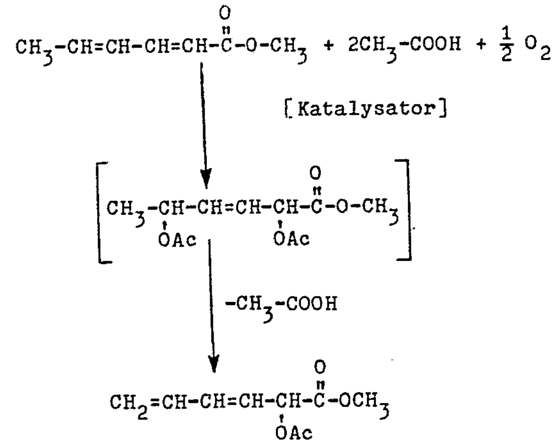


Es ist bekannt, dass sich 1-Acetoxy-1,3-butadiene mit Essigsäure und Sauerstoff in Gegenwart von Katalysoren, die Palladium und mindestens ein zusätzliches Element enthalten zu 1,1,4-Triacetoxy-2-butenen umsetzen lassen (DE-PS Nr. 2819592 und DE-OS Nr. 2842238).

Es ist weiterhin bekannt, dass bei der Umsetzung von 1-Acetoxy-1,3-butadien mit Essigsäure und Sauerstoff in Gegenwart von Palladium und Alkalisalze von Carbonsäuren enthaltenden Katalysatoren Gemische aus 1,4-Diacetoxy-2-buten und 3,4-Diacetoxy-1-buten entstehen (DE-OS Nr. 2200124). Hierbei wird also nur ein Molekül Essigsäure an eine Doppelbindung des 1,3-Diens angelagert.

Acyloxy

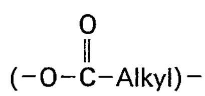

und Alkoxycarbonylgruppen

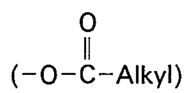

stellen zwar konstitutionsisomere Gruppen dar; ihr chemisches Verhalten unterscheidet sich jedoch wesentlich. So war nicht vorauszusehen, wie sich Alkadiencarbonsäureester bei der Umsetzung mit Sauerstoff und Carbonsäuren verhalten würden. In Analogie zur Reaktion von 1-Acetoxy-1,3-butadien mit Essigsäure und Sauerstoff hätte man erwarten können, dass sich z.B. bei der Behandlung von Sorbinsäuremethylester mit Essigsäure und Sauerstoff nicht 2-Acetoxy-3,5-hexadiencarbonsäuremethylester, sondern 2,5-Diacetoxy-3-hexencarbonsäuremethylester oder 5-Acetoxy-3-hexencarbonsäuremethylester bilden würden. Dass bei der erfindungsgemässen Umsetzung überwiegend 1,3-Dienderivate entstehen, war nicht vorauszusehen. Das Verfahren dieser Erfindung liefert die neuen Acyloxyalkadiencarbonsäureester auf einstufigem, besonders vorteilhaftem Wege.

Als Ausgangsstoffe der Formel II seien z.B. Methyl-, Ethyl-, Propyl-, Butyl-, Pentyl-, Cyclopentyl-, Cyclohexyl-, Benzyl- oder Phenylester der forgenden Säuren genannt: 2,4-Pentadiencarbonsäure, 3-Methyl-2,4-pentadiencarbonsäure, 4-Methyl-2,4-pentadiencarbonsäure, 3-n-Propyl-2,4-pentadiencarbonsäure, 4-tert.-butyl-2,4-pentadiencarbonsäure, 3,4-Dimethyl-2,4-pentadiencarbonsäure, 3,4-Di-n-butyl-2,4-pentadiencarbonsäure, 4-(2-Butenyl)-2,4-pentadiencarbonsäure, 3-(2-Pentenyl)-2,4-pentadiencarbonsäure, 2,4-Hexadiencarbonsäure, 2,4-Heptadiencarbonsäure, 2,4-Octadiencarbonsäure, 2,4-Nonadiencarbonsäure, 3-Methyl-2,4-hexadiencarbonsäure, 4-Ethyl-2,4-hexadiencarbonsäure, 2,4-Hexadiendicarbonsäure, 3-Methyl-2,4-hexadiendicarbonsäure und 3,4-Dimethyl-2,4-hexadiendicarbonsäure.

Die genannten Ausgangsverbindungen II können auf bekannte Weise hergestellt werden (Houben-Weyl, Methoden der organischen Chemie, 4. Auflage, Band V/1c, Seiten 1 bis 853). So ist Sorbinsäuremethylester z.B. aus Crotonaldehyd und Keten oder Malonsäure und 2,4-Pentadiencarbonsäuremethylester z.B. durch Abspaltung von zwei Molekülen Bromwasserstoffsäure aus 3,4-Dibrompentancarbonsäuremethylester zugänglich.

Als Carbonsäuren der Formel $R^7COOH$ kommen z.B. Ameisensäure, Essigsäure, Propionsäure, Buttersäuren oder Valeriansäuren in Betracht.

Als Katalysatoren verwendet man Palladium, Platin oder Salze dieser Metalle, wobei diese Katalysatoren noch andere aktive Bestandteile enthalten können. Beispielsweise sind Trägerkatalysatoren geeignet, die als aktive Bestandteile, welche auf dem Trägermaterial aufbracht sind, Palladium oder Platin und Kupfer und/oder Tellur enthalten. Die Katalysatoren lassen sich in bekannter Weise, z.B. nach Angaben der DE-PS Nr. 2217452, DE-OS Nrn. 2943407, 2820519 oder 2417452, 2820519 oder 2417558 herstellen. Katalysatoren der genannten Art enthalten beispielsweise, bezogen auf das Katalysatorgewicht, 1 bis 10% Palladium oder Platin, 0,1 bis 30% Kupfer und/oder 0,01 bis 10% Tellur. Bevorzugt ist die Verwendung eines Trägerkatalysators, der je Grammatom Palladium oder Platin 0,01 bis 6, vorzugsweise 1 bis 3,5-Grammatome Kupfer und/oder 0,01 bis 1, vorzugsweise 0,01 bis 0,4 Grammatome Tellur enthält. Die Gesamtmenge der auf den Träger aufgebrachten katalytisch wirksamen Metalle beträgt, bezogen auf den Trägerkatalysator, zweckmässig z.B. 0,01 bis 30% Gew.-%. Grössere und kleinere Konzentrationen sind allerdings auch möglich. Als Trägermaterial enthalten die Katalysatoren z.B. Aktivkohle, Bauxit, Bimsstein, Kieselgel, Kieselgur oder andere Formen der Kieselsäure, Magnesia, Ton oder Tonerde.

Man kann die katalytisch wirksamen Metalle, z.B. auch ohne Trägermaterial anwenden, indem man sie in Form von Salzen im Reaktionsgemisch löst oder suspendiert.

Die Umsetzung zur Herstellung der Verbindungen der Formel I nimmt man in an sich bekannter Weise in der Gas- oder Flüssigphase bei Temperaturen von 70 bis 180°C vor. In der Gasphase arbeitet man vorzugsweise bei 120 bis 150°C und in der Flüssigphase vorzugsweise bei 70 bis 110°C. Der Reaktionsdruck ist dadurch die Verfahrensweise gegeben und kann zwischen atmosphärischem Druck und z.B. 100 bar betragen. Das Verfahren kann chargenweise oder kontinuierlich, z.B. mit fixiertem Bett, Wirbelbett oder Dreiphasenfliessbett durchgeführt werden. Unumgesetzte Verbindungen der Formel II können nach der Umsetzung zusammen mit der jeweiligen Carbonsäure aus dem Reaktionsgemisch abdestilliert und z.B. in Form dieses Gemisches wieder eingesetzt werden.

Die erfindungsgemässen Acyloxyalkadiencarbonsäureester sind wertvolle Zwischenprodukte. Sie lassen sich z.B. mit Dienophilen durch Diels-Alder-Reaktion zu Cyclohexenderivaten umsetzen, die durch Alkyl-, Acetoxy- oder Alkoxycarbonylgruppen substituiert sind. Die Abspaltung von Essigsäure und/oder Wasserstoff führt zu verschiedenstens Benzol-, Phenol- oder Benzoesäurederivaten, die für die Herstellung von Farbstoffvorprodukten, Farbstoffen und Pharmazeutika von Bedeutung sind. So lässt sich bei der Umsetzung von 2-Acetoxy-3,5-hexadiencarbonsäuremethylester mit Ethylen und anschliessender Dehydrierung ein Mandelsäurederivat herstellen, aus dem durch Verseifung Mandelsäure oder durch Abspaltung von Essigsäure Zimtsäure entsteht. Aus 5-Acetoxy-2,4-pentadiencarbonsäuremethylester und Ethylen lässt sich aus dem zunächst entstehenden Cyclohexenderivat durch Abspaltung von Essigsäure und anschliessende Dehydrierung z.B. Benzoesäuremethylester herstellen.

*Beispiel 1*

In einen 1-Liter-Dreihalskolben, der mit Anschützaufsatz, Tropfrichter, Begasungsrührer, Innenthermometer, Gaseinleitungsrohr und Rückflusskühler mit aufgesetztem Trockeneiskühler versehen ist, werden nach dem Spülen der Apparatur mit Stickstoff 600 g Eisessig und 25 g eines nach den Angaben der DE-OS Nr. 2943407 hergestellten Pd/Cu/Te-Katalysators (5,1% Pd, 8,5% Cu, 0,9% Te) auf Aktivkohle als Träger eingetragen. Man erhitzt auf 95°C. Im Verlauf von 4 Stunden werden unter Rühren gleichzeitig 12 Liter Sauerstoff eingeleitet und 63 g Sorbinsäuremethylester zugetropft. Nach beendeter Zugabe leitet man bei 95°C in 30 Minuten 1,5 Liter Sauerstoff und anschliessend Stickstoff durch das Reaktionsgemisch. Man lässt abkühlen und entfernt den Katalysator durch Filtration. Bei der fraktionierten Destillation des Filtrats erhält man neben unumgesetztem Sorbinsäuremethylester 21 g 2-Acetoxy-3,5-hexadiencarbonsäuremethylester vom Siedepunkt 88 bis 115°C/25 mbar, $n_D^{20}$ = 1,4922.

*Beispiel 2*

56 g 2,4-Pentadiencarbonsäuremethylester werden, wie in Beispiel 1 beschrieben, in Gegenwart von 50 g eines nach den Angaben der DE-OS Nr. 2820519 hergestellten Pd/Cu-Katalysators (5%

Pd, 9,2% Cu) in 543 g Eisessig suspendiert und bei 95°C innerhalb von 4 Stunden mit 12 Litern Sauerstoff umgesetzt. Nach der in Beispiel 1 beschriebenen Aufarbeitung erhält man durch fraktionierte Destillation neben unumgesetztem 2,4-Pentadiencarbonsäuremethylester 13 g 2,5-Diacetoxy-3-pentencarbonsäuremethylester vom Siedepunkt 113 bis 115°C/0,4 mbar ($n_D^{20}$ = 1,4600) und 19,5 g 5-Acetoxy-2,4-pentadiencarbonsäuremethylester vom Siedepunkt 125 bis 131°C/13 mbar und Schmelzpunkt 54 bis 56°C (aus Methanol).

## Patentansprüche

1. Acyloxyalkadiencarbonsäureester der Formel

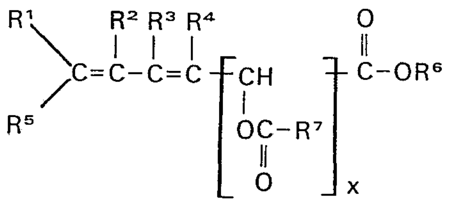 (I)

in der $R^1$ ein Wasserstoffatom, einen Kohlenwasserstoffrest mit 1 bis 5 C-Atomen oder einen $R^6$-O-CO-Rest, die Reste $R^2$ und $R^3$ Wasserstoffatome oder Kohlenwasserstoffreste mit 1 bis 5 C-Atomen, $R^4$ ein Wasserstoffatom oder einen Kohlenwasserstoffrest mit 1 bis 5 C-Atomen, jedoch für den Fall, dass x für O steht, ein Wasserstoffatom, $R^5$ ein Wasserstoffatom oder für den Fall, dass x für O steht, einen

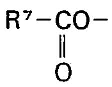 Rest,

$R^6$ einen Kohlenwasserstoffrest mit 1 bis 15 C-Atomen, $R^7$ ein Wasserstoffatom oder einen Kohlenwasserstoffrest mit 1 bis 5 C-Atomen und x entweder 0 oder 1 bedeuten.

2. Acyloxyalkadiencarbonsäureester nach Anspruch 1, dadurch gekennzeichnet, dass die Kohlenwasserstoffreste $R^1$, $R^2$, $R^3$, $R^4$, $R^6$ und $R^7$ in der Formel (I) Alkylreste mit 1 bis 3 C-Atomen bedeuten.

3. 2-Acetoxy-3,5-hexadiencarbonsäuremethylester.

4. 5-Acetoxy-2,4-pentadiencarbonsäuremethylester.

5. Verfahren zur Herstellung der Acyloxyalkadiencarbonsäureester nach Anspruch 1, dadurch gekennzeichnet, dass man Diene der Formel

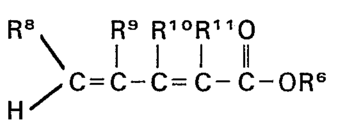 (II)

in der $R^8$ ein Wasserstoffatom, einen Kohlenwasserstoffrest mit 1 bis 5 C-Atomen oder einen $R^6$-O-CO-Rest und die Reste $R^9$, $R^{10}$ und $R^{11}$ Wasserstoffatome oder Kohlenwasserstoffreste mit 1 bis 5 C-Atomen bedeuten, in Gegenwart von Katalysatoren, die Palladium, Platin oder Salze dieser Metalle enthalten, und von Sauerstoff mit Carbonsäuren der Formel $R^7COOH$ behandelt.

## Claims

1. An acyloxyalkadienecarboxylate of the formula

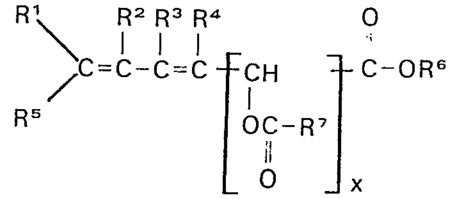 (I)

where $R^1$ is hydrogen, a hydrocarbon radical of 1 to 5 carbon atoms or $R^6$-O-CO-, $R^2$ and $R^3$ are each hydrogen or a hydrocarbon radical of 1 to 5 carbon atoms, $R^4$ is hydrogen or a hydrocarbon radical of 1 to 5 carbon atoms, but must be hydrogen if x is O, $R^5$ is hydrogen, or is

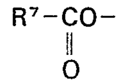

if x is O, $R^6$ is a hydrocarbon radical of 1 to 15 carbon atoms, $R^7$ is hydrogen or a hydrocarbon radical of 1 to 5 carbon atoms and x is 0 or 1.

2. An acyloxyalkadienecarboxylate as claimed in claim 1, wherein the hydrocarbon radicals $R^1$, $R^2$, $R^3$, $R^4$, $R^6$ and $R^7$ in formula (I) are each alkyl of 1 to 3 carbon atoms.

3. Methyl 2-acetoxy-3,5-hexadiencarboxylate.

4. Methyl 5-acetoxy-2,4-pentadienecarboxylate.

5. A process for the preparation of an acyloxyalkadienecarboxylate as claimed in claim 1, wherein a diene of the formula

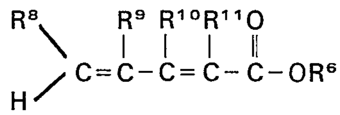 (II)

where $R^8$ is hydrogen, a hydrocarbon radical of 1 to 5 carbon atoms or $R^6$-O-CO-, and $R^9$, $R^{10}$ and $R^{11}$ are each hydrogen or a hydrocarbon radical of 1 to 5 carbon atoms, is treated with a carboxylic acid of the formula $R^7COOH$ in the presence of a catalyst containing palladium or platinum or a salt of one of these metals, and of oxygen.

## Revendications

1. Esters d'acides acyloxyalcadiène-carboxyliques de formule

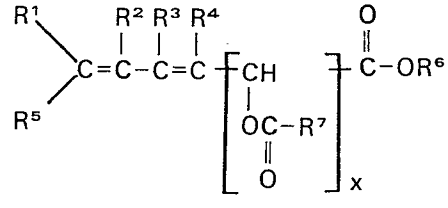 (I)

dans laquelle $R^1$ représente un atome d'hydrogène, un reste hydrocarboné en C1-C5 ou un reste $R^6$-O-CO-, les symboles $R^2$ et $R^3$ représentent des

atomes d'hydrogène ou des restes hydrocarbonés en C1-C5, $R^4$ représente un atome d'hydrogène ou un reste hydrocarboné en C1-C5, mais un atome d'hydrogène dans le cas où x = O, $R^5$ représente un atome d'hydrogène; ou bien lorsque x est égal à O, un reste

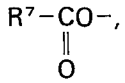

$R^6$ représente un reste hydrocarboné en C1-C15, $R^7$ un atome d'hydrogène ou un reste hydrocarboné en C1-C5 et x est égal à 0 ou 1.

2. Esters d'acides acyloxyalcadiène-carboxyliques selon la revendication 1, caractérisés en ce que les restes hydrocarbonés $R^1$, $R^2$, $R^3$, $R^4$, $R^6$ et $R^7$ de la formule (I) sont des restes alkyle en C1-C3.

3. Le 2-acétoxy-3,5-hexadiène-carboxylate de méthyle.

4. Le 5-acétoxy-2,4-pentadiène-carboxylate de méthyle.

5. Procédé de préparation des esters d'acides acyloxyalcadiène-carboxyliques selon la revendication 1, caractérisé en ce que l'on traite des diènes de formule

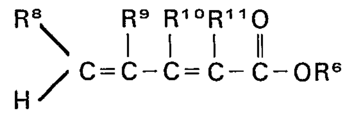

(II)

dans laquelle $R^8$ représente un atome d'hydrogène, un reste hydrocarboné en C1-C5 ou un reste $R^6$-O-CO- et les symboles $R^9$, $R^{10}$ et $R^{11}$ représentent des atomes d'hydrogène ou des restes hydrocarbonés en C1-C5, en présence de catalyseurs contenant du palladium, du platine ou des sels de ces métaux, et d'oxygène, par des acides carboxyliques de formule $R^7COOH$.